# EUROPEAN PATENT APPLICATION

(11) **EP 4 811 394 A1**
(43) Date of publication of application: **23.09.2026**
(21) Application number: 24908343.7
(22) Date of filing: 18.12.2024
(51) Int. Cl.: G16H 50/50, G16H 50/70, G16H 50/30, G16H 10/40, G16H 10/60, G16H 50/20, A61B 5/103, A61B 5/00

(54) **METHOD FOR BUILDING APHV ESTIMATION MODEL BASED ON BIOMETRIC DATA USING ARTIFICIAL INTELLIGENCE**

(30) Priority: 20.12.2023 KR 20230187450
(71) Applicant: GP Co., Ltd., Gwangmyeong-si, Gyeonggi-do 14316 (KR)
(72) Inventor: SEONG, Je Hyeok, Gwangmyeong-si Gyeonggi-do 14316 (KR); KIM, Ji Hun, Gwacheon-si Gyeonggi-do 13841 (KR); CHUN, Do Hyun, Seoul 02505 (KR)
(74) Representative: Isarpatent
(86) International application number: PCT/KR2024/097093
(87) International publication number: WO 2025/136039

(57) **Abstract**

A method for constructing a biometric data-based APHV estimation model using artificial intelligence according to exemplary embodiments of the present disclosure may include: receiving time-series biometric data of a plurality of sample subjects; estimating a plurality of first growth velocity curves each representing a growth velocity in height with respect to an actual age of a corresponding one of the plurality of sample subjects based on the time-series biometric data of the sample subjects; extracting, from each of the plurality of first growth velocity curves, first APHV (Age of Peak Height Velocity) data corresponding to an age at which the growth velocity reaches a maximum value; and training an artificial intelligence model using the first APHV data as target data.

## Description

### FIELD OF THE INVENTION

The present disclosure relates to a method, an apparatus, and a computer program for classifying growth stages based on APHV (Age of Peak Height Velocity) data, which indicates the age at which the growth velocity extracted from biometric data of growing children and adolescents reaches its maximum value, predicting growth-related characteristics, such as height, obesity, and diseases, and providing customized solutions for each growth stage.

### BACKGROUND ART

Recently, with the development of artificial intelligence technology, artificial intelligence technologies have been applied to various fields, and methods for generating additional information by extracting features inherent in data through neural network models, instead of conventional data processing methods, have been developed and utilized.

A neural network model used for artificial intelligence can, through learning, detect and recognize features in input data more quickly and accurately than conventional data processing. Recently, artificial intelligence technology has been applied not merely to tracking and detecting objects, but also to learning historical data and deriving current features that reflect future predictions or time-series changes.

Among these technologies, predictive analytics is a technology in the fields of statistics and data mining that extracts information from data and uses it to predict trends, behavioral patterns, and the like. Such predictive analytics may be applied to any field requiring decision-making based on information obtained from data. The core of predictive analytics lies in predicting unknown variables after understanding the relationships between variables.

To this end, various approaches are used depending on data characteristics and prediction targets.

The physical growth of adolescents is one of the various fields requiring predictive analytics. Parents, adolescents, and others have significant interest in when height growth will occur and how much height will be gained.

Conventionally, for predicting height growth, methods have been proposed for predicting growth by taking X-ray images of growth plates or by analyzing relationships with genetic/environmental factors (Korean Patent Registration Publication Nos. 10-2075743 and 10-1866208), and a method has also been proposed for converting physical data of sample subjects with different measurement timings or frequencies into a form suitable for training a growth prediction model (Korean Patent Registration Publication No. 10-2198302).

Although conventional methods for predicting the physical growth of children and adolescents have been proposed, as described above, research on methods for predicting the risk of precocious puberty, obesity, and the like, which may accompany the growth of children and adolescents, and for providing solutions thereto remains insufficient.

In addition, because children and adolescents undergo growth stages having distinct characteristics, these growth stages should be taken into consideration in order to improve the reliability of predicted data and of solutions provided through analysis.

Meanwhile, in the conventional technologies disclosed to date, various methods have been proposed in which physical data or biometric data of children and adolescents are directly input into a predetermined growth prediction tool to predict growth; however, according to such conventional technologies, growth is predicted solely based on general indicators, such as past and current heights, weights, BMIs, and the like, without taking into account individual growth-related characteristics, such as the growth velocity of each child or adolescent or whether a particular growth stage has been reached earlier relative to other sample subjects, and thus there is a disadvantage in that it is difficult to provide personalized solutions.

In addition, although the SITAR (SuperImposition by Translation And Rotation) methodology has been introduced in relation to height growth velocity, there is a problem in that only retrospective estimation is possible, because measurements taken throughout the entire growth period are required to estimate a growth velocity curve using the SITAR methodology.

Meanwhile, a conventional height growth prediction model predicts a height growth curve via an LGBM model using, as input variables, height, weight, body composition, percentiles thereof, and a benchmark growth rate based on a current percentile, and in a particular data set having a high proportion of observations with high height percentiles at older ages, an upward bias may occur in the estimation model. In addition, although the conventional height growth prediction model includes 52 body-composition-related variables, it utilizes only 8 variables mainly associated with height, weight, and body composition mass, and thus has a disadvantage in that the prediction accuracy of the estimation model is low.

Consequently, in the conventional height growth prediction model, although the growth velocity tendency of a sample subject is important for height prediction, information on past measurement values is not reflected in the estimation model, and thus it is necessary to measure a tracked growth velocity that reflects such past information.

### SUMMARY OF THE INVENTION

### [TECHNICAL PROBLEM]

One object of the present disclosure is to provide a method, an apparatus, and a computer program for classifying growth stages based on APHV (Age of Peak Height Velocity) data, which indicates the age at which the growth velocity of children and adolescents reaches its maximum value, and then predicting growth-related characteristics or conditions, such as height, obesity, and diseases, and providing customized solutions for each growth stage.

One object of the present disclosure is to provide a method, an apparatus, and a computer program for predicting growth-related characteristics or conditions, such as height, obesity, and diseases, based on the growth stages of children and adolescents by using an APHV estimation model constructed using artificial intelligence, and providing customized solutions for each growth stage.

### [SOLUTION]

A method for constructing a biometric data-based APHV estimation model using artificial intelligence according to exemplary embodiments of the present disclosure may include: receiving time-series biometric data of a plurality of sample subjects; estimating a plurality of first growth velocity curves each representing a growth velocity in height with respect to an actual age of a corresponding one of the plurality of sample subjects based on the time-series biometric data of the sample subjects; extracting, from each of the plurality of first growth velocity curves, first APHV (Age of Peak Height Velocity) data corresponding to an age at which the growth velocity reaches a maximum value; and training an artificial intelligence model using the first APHV data as target data.

The estimating of the first growth velocity curves may be performed by extracting age data and height data from the time-series biometric data of the sample subjects, and then applying the age data and the height data to a curve transformation model that uses the age data and the height data as input variables.

The curve transformation model may be a model constructed using a polynomial function.

The curve transformation model is a model constructed using Equation 1: ${y}_{it} = {α}_{i} + h \left(\frac{t - {β}_{i}}{exp \left(-{γ}_{i}\right)}\right)$

wherein αᵢ, βᵢ, and γᵢ are random correction values for adjusting a vertical shift, a horizontal shift, and a slope of a curve, respectively, h is a specific function related to height data, and t is age data.

The method for constructing a biometric data-based APHV estimation model using artificial intelligence according to exemplary embodiments of the present disclosure may further include, after the receiving of the time-series biometric data of the sample subjects, preprocessing the time-series biometric data of the sample subjects, wherein the preprocessing may be performed by determining, as noise, all biometric data of a sample subject for whom no biometric data collected during a specific growth period exists, and removing the biometric data.

The preprocessing may be performed by dividing growth periods of the plurality of sample subjects into a general growth period, a rapid growth period, and a decelerating growth period, and then removing, as noise, all data of a sample subject that does not have biometric data corresponding to each of the divided growth stages.

The preprocessing may be performed by further dividing the growth periods of the plurality of sample subjects into a slowed growth period and a growth-plate-closed period, and then removing, as noise, all data of a sample subject that does not have biometric data corresponding to each of the divided growth stages.

The preprocessing may be performed by dividing age ranges of the plurality of sample subjects into age groups from 8 years old to 18 years old, and then removing, as noise, all data of a sample subject for whom an input interval between adjacent biometric data entries is four years or more.

a sample subject for whom no biometric data entered during a period from 9 years old to 14 years old among the age ranges of the plurality of sample subjects exists.

### [EFFECT]

Since the biometric data-based APHV estimation model using artificial intelligence according to exemplary embodiments of the present disclosure estimates a growth velocity curve by applying a tracked growth velocity extracted from past information to a statistical methodology, extracts APHV (Age of Peak Height Velocity) data corresponding to an age at which the growth velocity reaches a maximum value from the growth velocity curve, and utilizes the APHV data in constructing various prediction models, such as an integrated height growth prediction model, a precocious puberty prediction model, and an obesity prediction model, the reliability of various prediction models, such as the integrated height growth prediction model, the precocious puberty prediction model, and the obesity prediction model, may be maximized.

In addition, according to various embodiments of the present disclosure, an accurate height may be predicted in consideration of the growth stages of children and adolescents through the APHV estimation model constructed through artificial intelligence learning, and solutions necessary for height growth may be provided in consideration of each growth stage.

### BRIEF DESCRIPTION OF THE DRAWINGS

FIG. 1 is a block diagram illustrating a configuration of a biometric data-based APHV estimation model using artificial intelligence according to exemplary embodiments of the present disclosure.
FIG. 2 is a graph illustrating predicted heights and target heights for respective growth stages according to exemplary embodiments of the present disclosure.
FIG. 3 is a flowchart illustrating a method for constructing a biometric data-based APHV estimation model using artificial intelligence according to exemplary embodiments of the present disclosure.
FIG. 4 is a graph illustrating a plurality of first growth velocity curves representing growth velocities in height with respect to actual ages based on time-series biometric data of sample subjects.
FIGS. 5 and 6 are tables illustrating APHV estimation errors according to age in months for girls and boys, respectively.
FIGS. 7 and 8 are graphs illustrating variable importance in APHV prediction for females and males, respectively.
FIG. 9 is a graph illustrating variable importance in a conventional growth prediction model for females and males, and FIG. 10 is a graph illustrating variable importance in a growth prediction model of the present disclosure.

### DETAILED DESCRIPTION OF THE PREFERRED EMBODIMENTS

Hereinafter, specific embodiments of the present disclosure will be described. The following detailed description is provided to assist in a comprehensive understanding of the method, apparatus, and/or system described in the present specification. However, this is merely exemplary, and the present disclosure is not limited thereto.

In describing embodiments of the present disclosure, when it is determined that a detailed description of known art related to the present disclosure may unnecessarily obscure the gist of the present disclosure, such detailed description will be omitted. In addition, the terms described below are terms defined in consideration of functions in the present disclosure, and may vary depending on the intention or practice of a user or operator. Therefore, the definitions thereof should be made based on the contents throughout the present specification. The terms used in the detailed description are merely for describing embodiments of the present disclosure and should in no way be limiting. Unless the context clearly indicates otherwise, expressions in the singular form include the meaning of the plural form. In the present description, expressions such as "comprising" or "including" are intended to indicate certain features, numbers, steps, operations, elements, parts thereof, or combinations thereof, and should not be construed as excluding the presence or possibility of one or more other features, numbers, steps, operations, elements, parts thereof, or combinations thereof in addition to those described.

In addition, in describing components of embodiments of the present disclosure, terms such as first, second, A, B, (a), and (b) may be used. These terms are used only to distinguish one component from another component, and the nature, sequence, order, or the like of the corresponding component is not limited by such terms.

In exemplary embodiments of the present disclosure, the term "children and adolescents" may be understood as encompassing a period during which the human body grows. More specifically, the term "children and adolescents," as used to refer to sample subjects in exemplary embodiments described below, is defined based on the meanings of infants, children, adolescents, young children, and pediatric-age children.

An infant is a person in a period continuing from the neonatal period up to two years after birth, during which the infant grows while suckling at the mother's breast, and experiences relating to nutrition, affection, and excretion during this period affect later general tendencies, and a child generally refers to a person in an age group of 6 years old or older and younger than 13 years old, and may also include a young child, who is 1 to 5 years old, in a broad sense.

An adolescent generally refers to a person who is 13 years old or older and younger than 19 years old based on age in completed years. A young child may refer to a person from one year after birth, or from 1 year old to 5 years old. A pediatric-age child may generally refer to a child up to 15 years old.

Accordingly, the term "children and adolescents," as used to refer to sample subjects, may, in a narrow sense, mean a period from 5 years old to 19 years old including children and adolescents, and may also, in a broad sense, mean a period including all general periods during which physical growth occurs, including the period from infancy to adolescence.

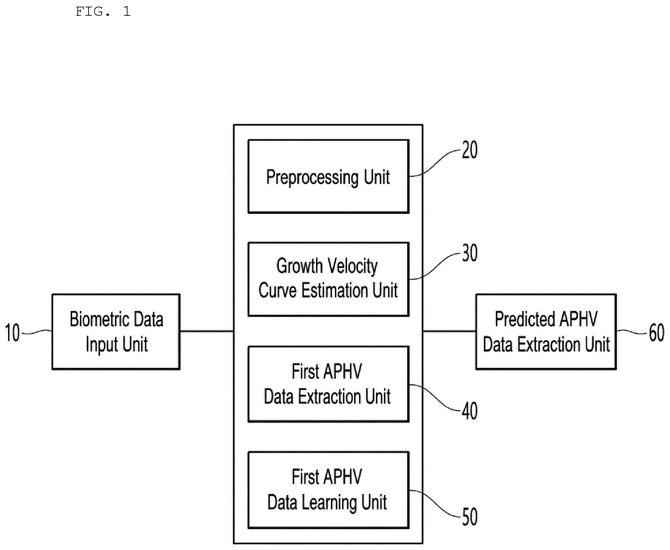

FIG. 1 is a block diagram illustrating a configuration of a biometric data-based APHV estimation model using artificial intelligence according to exemplary embodiments of the present disclosure.

Referring to FIG. 1, the biometric data-based APHV estimation model using artificial intelligence according to exemplary embodiments of the present disclosure may include a biometric data input unit (10), a preprocessing unit (20), a growth velocity curve estimation unit (30), a first APHV data extraction unit (40), and a first APHV data learning unit (50).

The biometric data-based APHV estimation model using artificial intelligence according to exemplary embodiments of the present disclosure may receive time-series physical information of a sample subject through the biometric data input unit (10). The physical information of the sample subject may include basic information, such as grade level, or age, gender, and height, as well as additional information, such as weight, protein, mineral content, body fat, body water, soft lean mass, fat-free mass, bone tissue, skeletal muscle mass, BMI (Body Mass Index), basal metabolic rate, neck circumference, chest circumference, abdominal circumference, thigh circumference, arm circumference, and hip circumference. Such physical information is merely an example provided to facilitate understanding of the present disclosure, the present embodiment is not limited thereto, and it is needless to say that the types of information constituting the physical information may be variously changed according to embodiments.

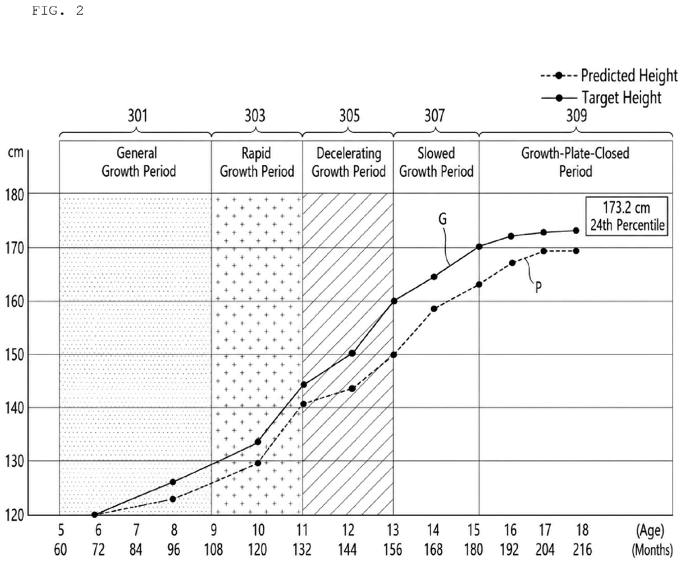

The time-series physical information of the sample subject may be continuous information or discontinuous information, but may be information included in at least one of the periods corresponding to the growth stages of FIG. 2.

More specifically, the time-series physical information of the sample subject may have various collection timings and collection frequencies. For example, in the case of a first sample subject, there may be physical information measured from 8 years old to 12 years old, which is a part of the period of children and adolescents, and in the case of a second sample subject, there may be physical information measured irregularly, such as at 8 years old, from 10 years old to 12 years old, and at 15 years old. In addition, in the case of a third sample subject, there may be physical information measured several times within a predetermined period, that is, within a period of any one of the plurality of growth stages of FIG. 2, whereas in the case of a fourth sample subject, there may be physical information measured only once within a predetermined period, that is, within a period of any one of the plurality of growth stages of FIG. 2.

As described above, according to the collection timing and the collection frequency, the physical information of the sample subject may be included in two or more of the growth stages of FIG. 2, as in the first sample subject and the second sample subject, but there may also be cases in which the physical information is not included in two or more of the growth stages of FIG. 2, as in the third sample subject and the fourth sample subject.

The preprocessing unit (20) may be configured to perform preprocessing on the time-series biometric data of the sample subjects.

In exemplary embodiments, the preprocessing unit (20) may perform the preprocessing by determining, as noise, all biometric data of a sample subject for whom no biometric data collected during a specific growth period exists, and removing such biometric data. This noise-removal process is intended to improve the prediction accuracy of the biometric data-based APHV estimation model according to the present disclosure and may not be applied when a sufficient amount of data has been accumulated; in such a case, the preprocessing may be performed by supplementing or additionally generating some biometric data through various methods, such as an artificial intelligence model or big data analysis, without determining all biometric data of a sample subject for whom no biometric data collected during a specific growth period exists to be noise and removing such biometric data.

The growth velocity curve estimation unit (30) may be configured to estimate a plurality of first growth velocity curves each representing a growth velocity in height with respect to an actual age of a corresponding one of the plurality of sample subjects based on the time-series biometric data of the sample subjects.

In exemplary embodiments, the estimation of the first growth velocity curves may be performed by extracting age data and height data from the time-series biometric data of the sample subjects, and then applying the age data and the height data to a curve transformation model that uses the age data and the height data as input variables.

In this case, the type of the curve transformation model is not particularly limited. That is, the curve transformation model may be a model constructed using a polynomial function, and, for example, the curve transformation model may be a model constructed using Equation 1.${y}_{it} = {α}_{i} + h \left(\frac{t - {β}_{i}}{exp \left(-{γ}_{i}\right)}\right)$

Here, αi, βi, and γi may be random correction values for adjusting a vertical shift, a horizontal shift, and a slope of a curve, respectively, h may be a specific function related to height data, and t may be age data.

However, the concept of the present disclosure is not necessarily limited thereto, and the curve transformation model may be a model constructed using a polynomial function other than Equation 1.

The first APHV data extraction unit (40) may be configured to extract, from each of the plurality of first growth velocity curves, first APHV (Age of Peak Height Velocity) data corresponding to an age at which the growth velocity reaches a maximum value.

The first APHV data may be used for diagnosing growth stages, constructing an estimation model, and deriving a growth age, and the growth age and the tracked growth velocity may ultimately be used for constructing various prediction models, such as an integrated height growth prediction model, a precocious puberty prediction model, and an obesity prediction model.

Meanwhile, a conventional height growth prediction model predicts a height growth curve via an LGBM model using, as input variables, height, weight, body composition, percentiles thereof, and a benchmark growth rate based on a current percentile, and in a particular data set having a high proportion of observations with high height percentiles at older ages, an upward bias may occur in the estimation model. In addition, although the conventional height growth prediction model includes 52 body-composition-related variables, it utilizes only 8 variables mainly associated with height, weight, and body composition mass, and thus has a disadvantage in that the prediction accuracy of the estimation model is low.

That is, in the conventional height growth prediction model, although the growth velocity tendency of a sample subject is important for height prediction, information on past measurement values is not reflected in the estimation model, and thus it is necessary to measure a tracked growth velocity that reflects past information. Therefore, the biometric data-based APHV estimation model using artificial intelligence according to exemplary embodiments of the present disclosure is characterized in that a growth velocity curve is estimated by applying a tracked growth velocity extracted from past information to a statistical methodology, APHV (Age of Peak Height Velocity) data corresponding to an age at which the growth velocity reaches a maximum value is extracted from the growth velocity curve, and the APHV data is then utilized in constructing various prediction models, such as an integrated height growth prediction model, a precocious puberty prediction model, and an obesity prediction model.

The first APHV data learning unit (50) may be configured to train an artificial intelligence model by inputting the first APHV data into the artificial intelligence model as target data.

Although the above description has been made based only on the biometric data input unit (10) receiving time-series physical information of a sample subject, the concept of the present disclosure is not necessarily limited thereto. That is, when there is a measurement subject for whom APHV estimation is desired through the APHV estimation model, the biometric data input unit (10) may be configured to further receive biometric data of the measurement subject.

In this case, the biometric data-based APHV estimation model using artificial intelligence according to exemplary embodiments of the present disclosure may further include a predicted APHV data extraction unit (60), and the predicted APHV data extraction unit (60) may be configured to extract predicted APHV (Age of Peak Height Velocity) data corresponding to an age at which the growth velocity of the measurement subject is expected to reach a maximum value by applying the biometric data of the measurement subject to an artificial intelligence model trained using, as target data, first APHV (Age of Peak Height Velocity) data corresponding to ages at which the growth velocities of the plurality of sample subjects reach maximum values.

FIG. 2 is a graph illustrating predicted heights and target heights for respective growth stages according to exemplary embodiments of the present disclosure.

Referring to FIG. 2 to examine the growth stages, the period of children and adolescents may include a general growth period (301), a rapid growth period (303), a decelerating growth period (305), a slowed growth period (307), and a growth-plate-closed period (309).

Each growth stage may be classified according to a degree of growth; the height gained each year may vary according to each growth stage, and even in the same growth stage, the actual height gained may vary according to a growth type.

The general growth period (301) generally refers to a period before the appearance of secondary sexual characteristics at puberty; children and adolescents in this period generally have open growth plates and, depending on the growth environment, grow by 4 to 5 cm per year in the case of a short-stature growth type and within a range of 6 to 7 cm per year in the case of a tall-stature growth type. In exemplary embodiments, the general growth period (301) may be defined as a period before a pubertal growth spurt of children and adolescents begins, and may be a growth stage having, as an end point, a time point preceding the predicted APHV by the first growth period. In one embodiment, the first growth period may be a period of one year to two years, for example, about 1.5 years.

The rapid growth period (303) is a period in which secondary sexual characteristics begin to appear, during which, in girls, the breasts swell and breast buds appear, and, in boys, the testicles enlarge, pubic hair begins to grow, and a voice change occurs. The rapid growth period (303) generally lasts for about 2 to 3 years after the general growth period (301), during which height increases, on average, within a range of about 7 to 10 cm per year. In exemplary embodiments, the rapid growth period (303) may be defined as a period during which a pubertal growth spurt begins and growth velocity increases until APHV, and may be a growth stage having, as a start point, a time point preceding the predicted APHV by the first growth period, and having the predicted APHV as an end point. In one embodiment, the first growth period may be a period of one year to two years, for example, about 1.5 years.

The decelerating growth period (305) refers to a period in which secondary sexual characteristics are being completed. In girls, this period may be identified based on menarche, and in boys, this period may be clearly identified based on physical changes such as the development of pubic hair, voice change, and the appearance of axillary hair. When the decelerating growth period (305) is reached, the degree of growth rapidly decreases compared to the rapid growth period (303); the decelerating growth period (305) generally lasts for about 2 to 3 years, during which height increases, on average, within a range of about 5 to 6 cm per year and natural growth comes to a stop. The growth plates begin to close gradually after the rapid growth period (303), and about 50% of the growth plates are closed about 6 months after entry into the decelerating growth period (305). In exemplary embodiments, the decelerating growth period (305) may be defined as a period during which growth velocity gradually decreases after APHV until the pubertal growth spurt ends, and may be a growth stage having the predicted APHV as a start point and having, as an end point, a time point following the predicted APHV by a second growth period. In one embodiment, the second growth period may be a period of one year to two years, for example, about 1.5 years.

The slowed growth period (307) may refer to a period during which growth velocity approaches 0 after the pubertal growth spurt and before entry into the growth-plate-closed period. In exemplary embodiments, the slowed growth period (307) may be defined as a period during which growth velocity gradually converges to 0 after the pubertal growth spurt of children and adolescents ends, and may be a growth stage having, as a start point, a time point following the predicted APHV by the second growth period and having, as an end point, a time point following the predicted APHV by a third growth period. In one embodiment, the third growth period may be a period of two years to four years, for example, about 3 years.

The growth-plate-closed period (309) refers to a period in which the growth plates are closed; in this period, although the growth period has not completely ended, natural height growth has become difficult. In general, girls enter the growth-plate-closed period (309) after about 1 year and 6 months to 2 years from menarche, and boys enter the growth-plate-closed period (309) after about 1 year and 6 months to 2 years from the time point at which axillary hair appears. In the growth-plate-closed period (309), the growth plates are closed and natural growth stops, but growth within a range of about 1 to 3 cm may still occur through improvement of physical functions by changing improper lifestyle habits, customized exercise, posture correction, nutrient intake, and the like. In exemplary embodiments, the growth-plate-closed period (309) may be defined as a period during which growth velocity is close to 0 after the growth plates are closed, and may be a growth stage having, as a start point, a time point following the predicted APHV by the third growth period. In one embodiment, the third growth period may be a period of two years to four years, for example, about 3 years.

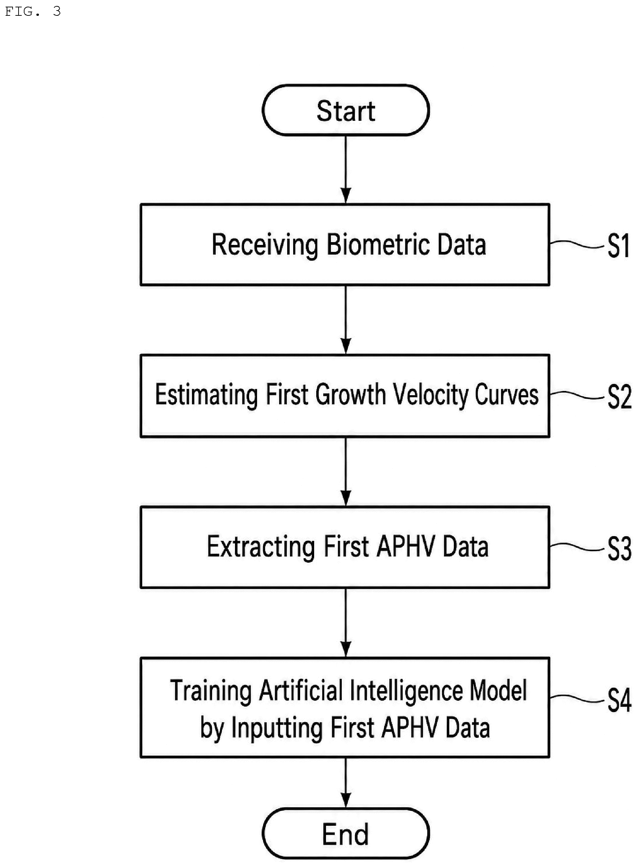

FIG. 3 is a flowchart illustrating a method for constructing a biometric data-based APHV estimation model using artificial intelligence according to exemplary embodiments of the present disclosure.

Referring to FIG. 3, the method for constructing a biometric data-based APHV estimation model using artificial intelligence according to exemplary embodiments of the present disclosure may include receiving time-series biometric data of a plurality of sample subjects (S1), estimating a plurality of first growth velocity curves each representing a growth velocity in height with respect to an actual age of a corresponding one of the plurality of sample subjects based on the time-series biometric data of the sample subjects (S2), extracting, from each of the plurality of first growth velocity curves, first APHV (Age of Peak Height Velocity) data corresponding to an age at which the growth velocity reaches a maximum value (S3), and training an artificial intelligence model by inputting the first APHV data into the artificial intelligence model as target data (S4).

The receiving of the time-series biometric data (S1) may be performed by receiving time-series physical information of a sample subject through the biometric data input unit (10).

The estimating of the first growth velocity curves (S2) may be performed by extracting age data and height data from the time-series biometric data of the sample subjects, and then applying the age data and the height data to a curve transformation model that uses the age data and the height data as input variables.

In exemplary embodiments, the curve transformation model may be a model constructed using a polynomial function.

In one embodiment, the curve transformation model may be a model constructed using Equation 1.${y}_{it} = {α}_{i} + h \left(\frac{t - {β}_{i}}{exp \left(-{γ}_{i}\right)}\right)$

In Equation 1, αi, βi, and γi may be random correction values for adjusting a vertical shift, a horizontal shift, and a slope of a curve, respectively, h may be a specific function related to height data, and t may be age data.

The method for constructing a biometric data-based APHV estimation model using artificial intelligence according to exemplary embodiments of the present disclosure may further include, after the receiving of the time-series biometric data of the sample subjects, preprocessing the time-series biometric data of the sample subjects (not shown).

The preprocessing may be performed by determining, as noise, all biometric data of a sample subject for whom no biometric data collected during a specific growth period exists, and removing such biometric data.

In exemplary embodiments, the preprocessing may be performed by dividing the growth periods of the plurality of sample subjects into a general growth period, a rapid growth period, and a decelerating growth period, and then removing, as noise, all data of a sample subject that does not have biometric data corresponding to each of the divided growth stages.

In one embodiment, the preprocessing may be performed by further dividing the growth periods of the plurality of sample subjects into a slowed growth period and a growth-plate-closed period, and then removing, as noise, all data of a sample subject that does not have biometric data corresponding to each of the divided growth stages.

For example, the preprocessing may be performed by dividing the age ranges of the plurality of sample subjects into age groups from 8 years old to 18 years old, and then removing, as noise, all data of a sample subject for whom an input interval between adjacent biometric data entries is four years or more.

Alternatively, the preprocessing may be performed by removing, as noise, all data of a sample subject for whom no biometric data was entered during a period from 9 years old to 14 years old among the age ranges of the plurality of sample subjects.

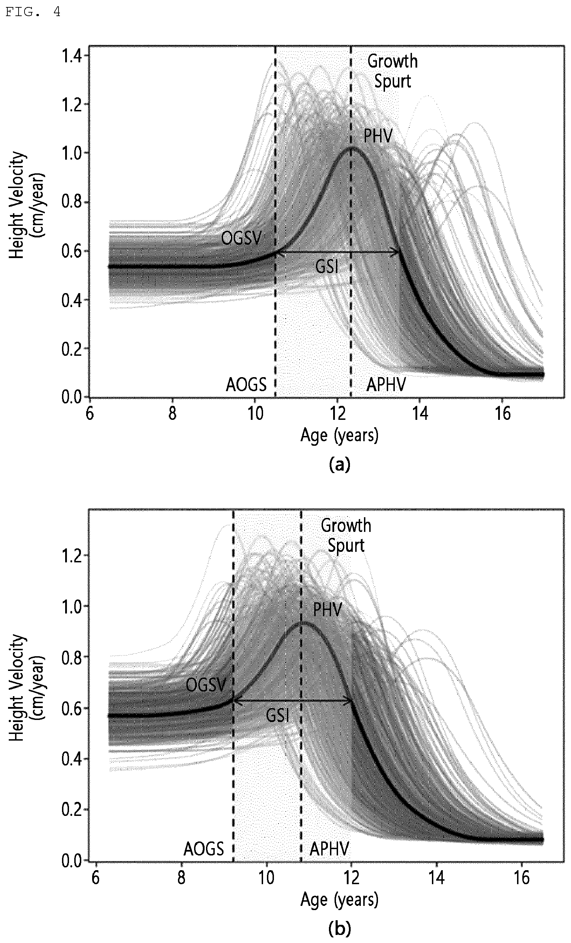

FIG. 4 is a graph illustrating a plurality of first growth velocity curves representing growth velocities in height with respect to actual ages based on time-series biometric data of sample subjects.

FIG. 4 illustrates a plurality of first growth velocity curves generated through the growth velocity curve estimation unit (30), and the first APHV data extraction unit (40) may be configured to extract, from each of the plurality of first growth velocity curves, first APHV (Age of Peak Height Velocity) data corresponding to an age at which the growth velocity reaches a maximum value. The first APHV data may refer to data representing the age at which, during a pubertal growth spurt, growth velocity reaches its maximum value, that is, the age at which growth acceleration is 0.

Meanwhile, for each of the plurality of first growth velocity curves, the first APHV data extraction unit (40) may also be configured to further extract additional data in addition to first APHV (Age of Peak Height Velocity) data corresponding to an age at which the growth velocity reaches a maximum value.

For example, the first APHV data extraction unit (40) may be configured to further extract AOGS (Age of Onset of Growth Spurt) data corresponding to a point at which a pubertal growth spurt begins, AEGS (Age of End of Growth Spurt) data corresponding to a point at which the pubertal growth spurt ends, and the like.

The AOGS data may refer to data corresponding to a point at which the slope of the growth velocity sharply increases and the growth acceleration reaches a maximum value, and may represent an age about 1.5 years before the age indicated by the first APHV data.

The AEGS data may refer to data corresponding to a point at which the pubertal growth spurt ends, that is, a point at which the growth velocity after APHV becomes equal to the growth velocity at AOGS (onset-of-growth-spurt velocity), and may represent an age about 1.5 years after the age indicated by the first APHV data.

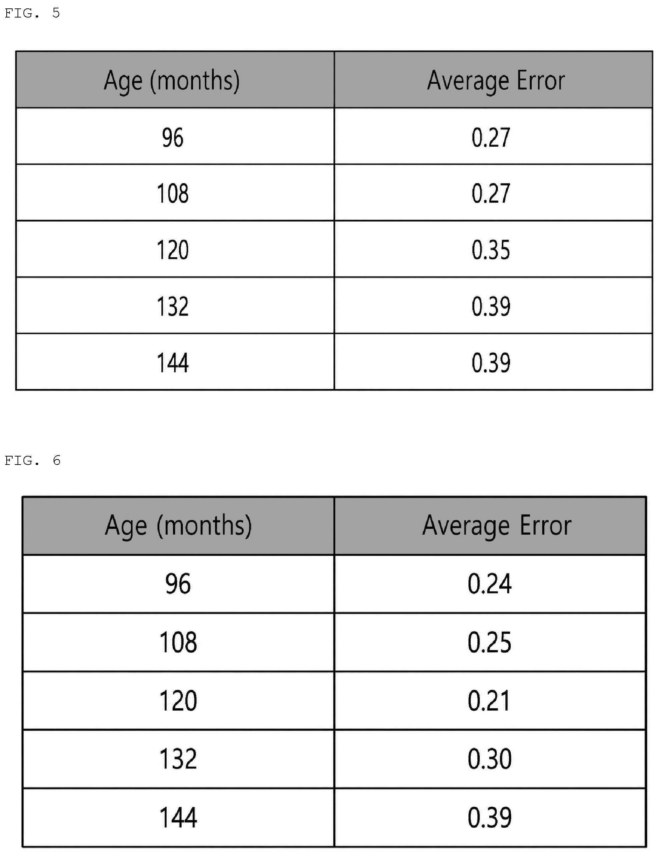

FIGS. 5 and 6 are tables illustrating APHV estimation errors according to age in months for girls and boys, respectively.

Referring to FIGS. 5 and 6, predicted APHV data of measurement subjects of various ages were extracted using the biometric data-based APHV estimation model using artificial intelligence according to exemplary embodiments of the present disclosure, and then the predicted APHV data were compared with actual APHV data of the measurement subjects.

Specifically, through the above experimental results, it was confirmed that, in the case of girls aged 10 years or older, the average error between the predicted APHV data values and the actual APHV data values was 0.35 years, and, in the case of boys aged 12 years or older, the average error between the predicted APHV data values and the actual APHV data values was 0.39 years. That is, as a result, it was confirmed that, in the case of the biometric data-based APHV estimation model using artificial intelligence according to exemplary embodiments of the present disclosure, for both girls and boys, the average error between the predicted data values and the actual data values at the average age range in which secondary sexual characteristics appear was within 0.5 years, and thus the APHV prediction accuracy was very high.

In addition, through the above experiment, it was also confirmed that the prediction accuracy of the APHV estimation model improves as the actual ages of the measurement subjects become closer to the ages indicated by the actual APHV data.

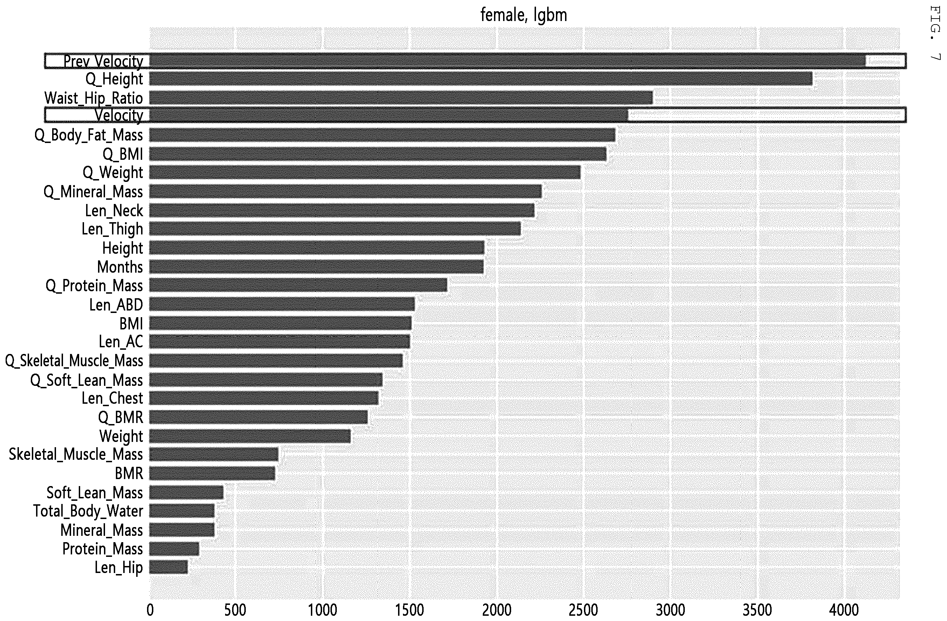

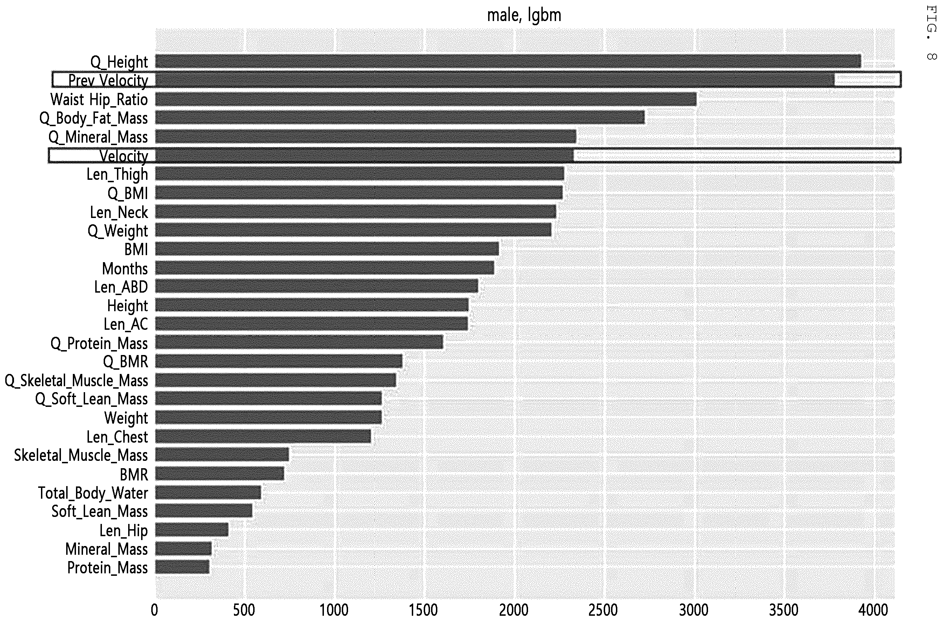

FIGS. 7 and 8 are graphs illustrating variable importance in APHV prediction for females and males, respectively.

Referring to FIGS. 7 and 8, various variables for constructing the biometric data-based APHV estimation model using artificial intelligence according to exemplary embodiments of the present disclosure were classified according to importance.

Specifically, as illustrated in FIG. 7, the variable having the highest importance in APHV prediction for females was previous growth velocity (Previously Velocity), whereas, as illustrated in FIG. 8, the variable having the highest importance in APHV prediction for males was height quantile (Height Quantile).

Meanwhile, in APHV prediction for females, current growth velocity (Velocity) was found to be the variable having the fourth-highest importance, and in APHV prediction for males, previous growth velocity (Previously Velocity) and current growth velocity (Velocity) were found to be the variables having the second-highest and sixth-highest importance, respectively.

That is, it was confirmed that previous growth velocity (Previously Velocity) and current growth velocity (Velocity) each serve as a very important variable in APHV prediction for females and males; in particular, previous growth velocity (Previously Velocity) was found to be the most important variable for females and the second-most important variable for males. As a result, it was confirmed that the accuracy of predicted APHV data extracted using the APHV estimation model may be greatly improved when two or more pieces of biometric data measured at different time points exist for a measurement subject, thereby enabling previous growth velocity to be identified.

In addition, it was confirmed that, in order to improve the accuracy of the predicted APHV data extracted through the biometric data-based APHV estimation model using artificial intelligence according to exemplary embodiments of the present disclosure, additional variables, such as height quantile (Height Quantile), waist-to-hip ratio (Waist to Hip Ratio; WHR), and body fat mass (Body Fat Mass), are required.

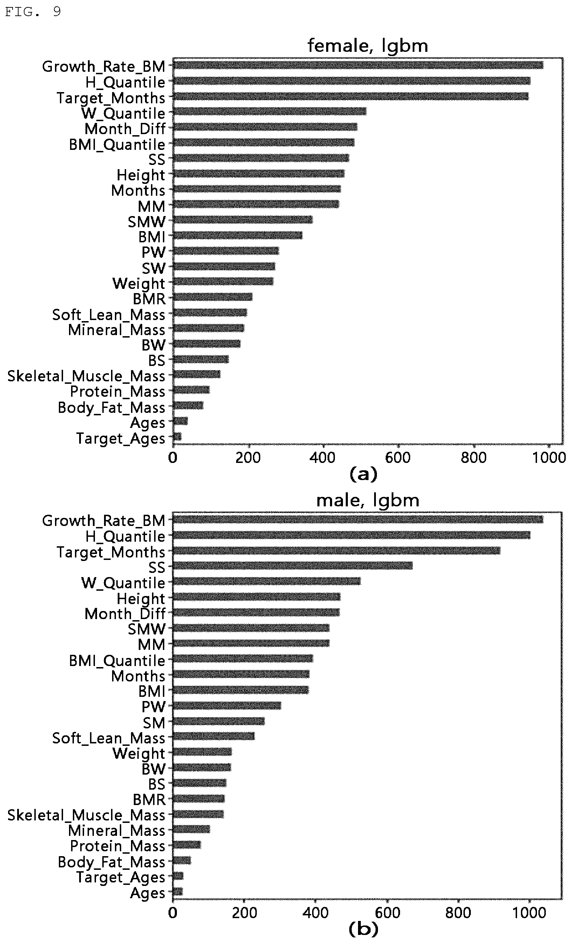

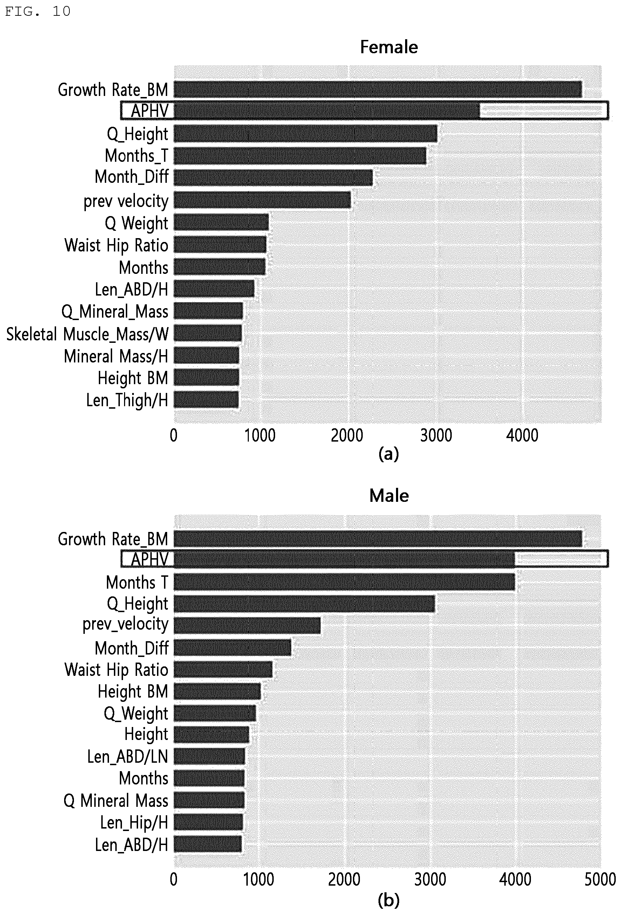

FIG. 9 is a graph illustrating variable importance in conventional growth prediction models for females and males, and FIG. 10 is a graph illustrating variable importance in a growth prediction model of the present disclosure.

Referring to FIGS. 9 and 10, when the conventional growth prediction models for females and males were compared with the growth prediction model of the present disclosure, the variable having the highest importance was found to be the same in all models, namely growth rate (Growth Rate); however, in the conventional growth prediction models, the variable having the second-highest importance was height quantile (Height Quantile), and, in the growth prediction model of the present disclosure, the variable having the second-highest importance was APHV (Age of Peak Height Velocity).

That is, in the conventional growth prediction model before the concept of APHV was applied, height quantile (Height Quantile) was found to be the most important variable after growth rate (Growth Rate), whereas, in the growth prediction model according to the present disclosure after the concept of APHV (Age of Peak Height Velocity) was applied, APHV (Age of Peak Height Velocity) was calculated as having greater importance than height quantile (Height Quantile) and was found to be the most important variable after growth rate (Growth Rate); accordingly, it was confirmed that APHV has very high importance among variables affecting predicted final height.

As described above, since the biometric data-based APHV estimation model using artificial intelligence according to exemplary embodiments of the present disclosure estimates a growth velocity curve by applying a tracked growth velocity extracted from past information to a statistical methodology, extracts APHV (Age of Peak Height Velocity) data corresponding to an age at which the growth velocity reaches a maximum value from the growth velocity curve, and then utilizes the APHV data in constructing various prediction models, such as an integrated height growth prediction model, a precocious puberty prediction model, and an obesity prediction model, the reliability of various prediction models, such as the integrated height growth prediction model, the precocious puberty prediction model, and the obesity prediction model, may be maximized.

In addition, according to various embodiments of the present disclosure, height may be accurately predicted in consideration of the growth stages of children and adolescents through an APHV estimation model constructed through artificial intelligence learning, and solutions necessary for height growth may be provided in consideration of each growth stage.

However, the concept of the present disclosure is not necessarily limited thereto, and the apparatus, method, or system according to exemplary embodiments of the present disclosure may be applied to various products and technical fields in addition to the above-described products and technical fields.

Although various embodiments of the present disclosure have been described in detail above, those of ordinary skill in the art to which the present disclosure pertains will understand that various modifications may be made to the above-described embodiments within the scope of the present disclosure. Therefore, the scope of the present disclosure should not be limited to the described embodiments, but should be defined by the following claims as well as equivalents thereof.

## Claims

1. A method for constructing a biometric data-based APHV estimation model using artificial intelligence, the method comprising:
receiving time-series biometric data of a plurality of sample subjects;
estimating a plurality of first growth velocity curves each representing a growth velocity in height with respect to an actual age of a corresponding one of the plurality of sample subjects based on the time-series biometric data of the sample subjects;
extracting, from each of the plurality of first growth velocity curves, first APHV (Age of Peak Height Velocity) data corresponding to an age at which the growth velocity reaches a maximum value; and
training an artificial intelligence model using the first APHV data as target data.

2. The method of claim 1, wherein the estimating of the plurality of first growth velocity curves is performed by extracting age data and height data from the time-series biometric data of the sample subjects, and then applying the age data and the height data to a curve transformation model that uses the age data and the height data as input variables.

3. The method of claim 2, wherein the curve transformation model is a model constructed using a polynomial function.

4. The method of claim 3, wherein the curve transformation model is a model constructed using Equation 1: ${y}_{it} = {α}_{i} + h \left(\frac{t - {β}_{i}}{exp \left(-{γ}_{i}\right)}\right)$ wherein αᵢ, βᵢ, and γᵢ are random correction values for adjusting a vertical shift, a horizontal shift, and a slope of a curve, respectively, h is a specific function related to height data, and t is age data.

5. The method of claim 1, further comprising, after the receiving of the time-series biometric data of the sample subjects, preprocessing the time-series biometric data of the sample subjects,
wherein the preprocessing is performed by determining, as noise, all biometric data of a sample subject for whom no biometric data collected during a specific growth period exists, and removing the biometric data.

6. The method of claim 5, wherein the preprocessing is performed by dividing growth periods of the plurality of sample subjects into a general growth period, a rapid growth period, and a decelerating growth period, and then removing, as noise, all data of a sample subject that does not have biometric data corresponding to each of the divided growth stages.

7. The method of claim 6, wherein the preprocessing is performed by further dividing the growth periods of the plurality of sample subjects into a slowed growth period and a growth-plate-closed period, and then removing, as noise, all data of a sample subject that does not have biometric data corresponding to each of the divided growth stages.

8. The method of claim 5, wherein the preprocessing is performed by dividing age ranges of the plurality of sample subjects into age groups from 8 years old to 18 years old, and then removing, as noise, all data of a sample subject for whom an input interval between adjacent biometric data entries is four years or more.

9. The method of claim 8, wherein the preprocessing is performed by removing, as noise, all data of a sample subject for whom no biometric data entered during a period from 9 years old to 14 years old among the age ranges of the plurality of sample subjects exists.

10. The method of claim 6, wherein the general growth period is defined as a period before a pubertal growth spurt of children and adolescents begins,
wherein the general growth period is a growth stage having, as an end point, a time point preceding the first APHV by a first growth period.

11. The method of claim 10, wherein the first growth period is a period of one year to two years

12. The method of claim 11, wherein the rapid growth period is defined as a period in which secondary sexual characteristics begin to appear in children and adolescents,
wherein the rapid growth period is a growth stage having, as a start point, a time point preceding the first APHV by the first growth period, and having the first APHV as an end point.

13. The method of claim 11, wherein the decelerating growth period is defined as a period in which secondary sexual characteristics of children and adolescents are being completed,
wherein the decelerating growth period is a growth stage having the first APHV as a start point and having, as an end point, a time point following the first APHV by a second growth period.

14. The method of claim 13, wherein the second growth period is a period of one year to two years.

15. The method of claim 14, wherein the first growth period and the second growth period are set to be different periods.
